# EUROPEAN PATENT SPECIFICATION

(11) **EP 2 015 759 B1**
(45) Date of publication and mention of the grant of the patent: **04.08.2010**
(21) Application number: 07735778.8
(22) Date of filing: 04.05.2007
(51) Int. Cl.: A61K 31/7072, A61P 31/18, A61K 45/06

(54) **COMBINATION THERAPY**
KOMBINATIONSTHERAPIE
THÉRAPIE EN COMBINAISON

(30) Priority: 05.05.2006 GB 0608876
(43) Date of publication of application: 21.01.2009
(73) Proprietor: Medivir AB, 141 44 Huddinge (SE)
(72) Inventor: ZHANG, Hong, 141 44 Huddinge (SE); OBERG, Bo, 141 44 Huddinge (SE)
(74) Representative: Teuten, Andrew John
(86) International application number: PCT/IB2007/051688
(87) International publication number: WO 2007/129274

(56) References cited:
- WO-A1-91/01137
- COX S W ET AL: "Loss of synergistic response to combinations containing AZT in AZT- resistant HIV-1" AIDS RESEARCH AND HUMAN RETROVIRUSES 1992 US, vol. 8, no. 7, 1992, pages 1229-1234, XP009118622 ISSN: 0889-2229
- COX S.: 'Metabolism of 3'-azido-3'-deoxythymidine and 3'-fluoro-3'-deoxythymidine in combination in human immunodeficienc virus infected lymphoblastoid cells' ANTIVIRAL CHEMISTRY & CHEMOTHERAPY vol. 3, no. 3, 1992, pages 165 - 170, XP003018683
- HARMENBERG J. ET AL.: 'Synergistic Inhibition of Human Immunodeficiency Virus Replication In Vitro by Combinations of 3'-Azido-3'-Deoxythymidine and 3'-Fluoro-3'-Deoxythymidine' AIDS RESEARCH AND HUMAN RETROVIRUSES vol. 6, no. 10, 1990, pages 1197 - 1202, XP003018684

## Description

### Technical field

This invention relates to HIV treatment and pharmaceutical compositions wherein the known HIV antivirals alovudine and zidovudine are administered in a specified ratio, optionally in combination with further antivirals.

### Background Art

Zidovudine, also known as AZT or 2'3'-dideoxy-3'-azido-thymidine, was the first nucleoside analogue registered for use in the treatment of HIV. Zidovudine inhibits the virally encoded reverse transcriptase enzyme, thereby blocking the viral replication cycle and effectively slowing the progression of AIDS. When first introduced and prescribed as long term monotherapy, zidovudine was associated with various tissue pathologies featuring the symptoms of mitochondrial dysfunction, including skeletal muscle myopathy, dilated cardiomyopathy and hepatoxicity. Although still widely used in conjunction with other HIV antivirals, toxicity, in particular hematological toxicity, and resistance development issues limit the clinical use of zidovudine. The current administration regime is twice daily dosing, typically 300 mg BID (i.e. a daily adult dose of 600 mg taken as separate 300 mg tablets morning and night).

Even combination unit dosage forms such as Combivir™ (zidovudine plus lamivudine) or Trizivir™ (zidovudine plus lamivudine plus abacavir) must be taken BID. This is not a convenient dosing regime at the best of times from a patient compliance viewpoint. It is however worse with HIV patients since many patients prescribed zidovudine are required to take still further HIV antiviral drugs such as protease inhibitors and/or non-nucleoside inhibitors or symptomatic medications such as antifungals, anti-CMV antivirals, antipsychotics or immune stimulators. These additional pharmaceuticals are often taken at a different periodicity (QD, TID etc) to zidovudine leading to very complex pill regimes with poor compliance. For example it is not uncommon that advanced HIV patients have a pill burden in excess of 15-20 tablets per day, at various time points during the day, some fasting and some with meals, and with a varying number of pills at each timepoint.

Lack of compliance with dosing regimes is of crucial importance in the case of HIV where drug escape mutants readily arise due to the poor proofreading capacity of the HIV reproductive machinery. The selection and propagation of drug escape mutants is dramatically accelerated if serum trough levels of the HIV medications and intracellular levels of nucleoside triphosphates fall below a certain threshold. This quickly happens in HIV patients if the prescribed dosage regime is not exhaustively followed.

Alovudine (also known as FLT or 2',3'-dideoxy-3'-fluorothymidine) was a promising HIV antiviral in clinical development during the early 1990s. Its development was terminated after dose-dependent haematologic toxicity was observed in virus infected patients. On the basis of cell culture experiments, this haemopoetic toxicity was attributed by Sundseth et al. Antmicrob Ag Chemother 1996 40(2):331-335 to DNA fragmentation and apoptosis. However, recent studies reveal that the toxicity of alovudine is due to the inhibition of mitochondrial DNA synthesis.

International patent application WO91/01137 which was filed before the original development of alovudine was terminated, describes a synergistic antiviral effect of combinations of 3'-fluorinated antivirals such as FLT (now known by the INN alovudine) and certain 2',3'-dideoxy nucleotides including AZT (now known by the INN zidovudine). The patent application exemplifies combinations with a ratio of AZT:FLT of 1:1 and 8:1 in *in vitro* and animal experiments. The patent application makes it clear that it is preferred that the FLT and AZT are administered in substantially equal amounts. For example page 5 line 16 of the patent application indicates that the preferred range of FLT to AZT giving a synergistic effect is 10:1 to 1:20, with the optimal range being 1:1 to 1:10 FLT:AZT. Table 1 of WO91/01137 indicates that the FLT:AZT in the ratio 1:1 had a superior therapeutic index, IC₅₀/IC₅₀ relative to the FLT:AZT 1:8 ratio as measured by an immunofluorescence assay and a comparable therapeutic index as measured by ELISA.

International patent application W02004/002433 describes an alternative combination of alovudine and another NRTI, namely abacavir. The synergistic activity as regards viral reduction was identified in clinical trials on HIV infected patients where the patients were prescribed 7.5 mg QD alovudine as an add-on to existing antiretroviral treatments. The study design and overall results are outlined in Katlama et al. AIDS 2004 18(9):1299-1304. Note in particular that patients already being treated with zidovudine were excluded, as it was felt that the close structural similarity of alovudine and zidovudine could result in an additional haematological toxicity. Abacavir is typically administered 300 mg BID or 600 mg QD. This corresponds to a molar ratio of 1:70 alovudine to abacavir. The antiviral synergy was confirmed in the patent specification in cell culture at a molar ratio of 1:200. However, neither cellular nor mitochondrial toxicity of the combination was measured. As shown in the comparative examples herein, alovudiine:abacavir at a molar ratio intermediate these values is not able to reverse the mitochondrial toxicity of alovudine.

The interaction of nucleoside reverse transcriptase inhibitors, especially as regards mitochondrial toxicity is a complex and poorly understood phenonoma. In a very comprehensive series of studies reported in Vidal et al. Antimicrob Ag Chemother 2006 50(11):3824-3832, the NRTI tenofovir was shown to dramatically enhance the mitochondrial toxicity (as measured by mtDNA depletion) of didanosine (ddl). At dosages of 3 uM didanosine : 30 uM tenofovir (i.e. 1:10 on a molar basis) the reduction in mtDNA was approximately 80% and >90% at higher molar ratios. In contrast tenofovir did not affect the mitochondrial toxicity of zidovudine when tested at 3, 40 and 200 uM zidovudine to 30 uM tenofovir (i.e. 1:10, around 1:1 and around 6.5:1).

### Brief description of the invention

We have now discovered that co-administration of alovudine and zidovudine at a particular range of ratios well outside those of the prior art produce an interaction with surprisingly reduced mitochondrial toxicity, while retaining the synergistic antiviral efficacy of alovudine and zidovudine.

The present application discloses a method for the treatment or prophylaxis of HIV comprising the simultaneous or sequential administration of alovudine and zidovudine at a molar ratio in the range 1:100 to 1:350. Suitably the method comprises the administration of a safe and effective amount of alovudine and zidovudine, to a subject in need thereof, thereby to treat or prevent HIV.

Consequently, in a first aspect of the invention there is provided alovudine for use in the treatment or prevention of HIV with simultaneous or sequential administration of zidovudine at a molar ratio of alovudine to zidovudine in the range 1:100 to 1:350.

Additionally provided is zidovudine for use in the treatment or prevention of HIV with simultaneous or sequential administration of alovudine at a molar ratio of alovudine to zidovudine in the range 100:1 to 350:1.

A related aspect of the invention provides the use of alovudine and zidovudine in the manufacture of medicaments for simultaneous or sequential administration, whereby the medicaments are adapted to encourage dosing in the ratio 1:100 to 1:350, alovudine to zidovudine.

A second aspect of the invention provides a pharmaceutical composition adapted for use in the method and comprising alovudine and zidovudine at a ratio in the molar range 1:100 to 1:350 and wherein the alovudine is present in the range 2-4 mg and the zidovdine is present in the range 300-900 mg.

Accordingly, there is also provided a kit of parts comprising a pharmaceutical composition comprising alovudine and a pharmaceutical composition comprising zidovudine, characterized in that that the alovudine and zidovudine are present in the kit at a molar ratio in the range 1:100 to 1:350. Suitably the kit of parts will additionally comprise instructions directing the simultaneous or sequential administration of the pharmaceutical composition comprising alovudine and the pharmaceutical composition comprising zidovudine for the treatment or prevention of HIV.

The combinations of the invention alleviate shortcomings experienced with prior art zidovudine and alovudine treatments and zidovudine/alovudine combinations notably in regard to decreased mitochondrial toxicity and thus improved safety, better patient compliance, improved consistency of daily trough levels and reduced drug escape mutant breakthrough.

Although not wishing to be bound by theory, our preliminary data suggests that adoption of the characteristic ratio between alovudine and zidovudine allows zidovudine to interfere with mitochondrial transport mechanisms thereby preventing the active metabolite alovudine triphosphate from negatively interacting with the especially sensitive mitochondrial DNA polymerase. This beneficial effect was not previously seen in prior art AZT/FLT combinations as it was masked by the cellular toxicity and reduction in mitochondrial DNA induced by the substantially equimolar amounts of the two nucleosides.

In contrast to the prior art combinations of WO91/01137 which are predicated on alovudine dosages of 0.1 to 1 mg/kg/day in conjunction with zidovudine dosages of 1-10 mg/kg/day, the present invention envisages alovudine dosages of the order of 0.005 to 0.05 mg/kg/day in conjunction with the corresponding dose of zidovudine 1-10 mg/kg/day. More recent clinical studies have suggested that the 1:10 ratio preferred in WO91/01137 based on the effective dose of zidovudine leads to a toxic level of alovudine and/or zidovudine.

Conveniently the compositions and methods of the invention employ alovudine and zidovudine at a ratio in the range 1:150 to 1:250, such as within the range 1:150 to 1:200.

Typically the maximum daily dosage of alovudine will be of the order of 4 mg/day for a 70 kg adult. Dosage regimes in accordance with the method of the invention will thus generally include an alovudine dosage in the range 2-4 mg per day and a zidovudine dosage in the rage 300-900 mg per day.

Particularly preferred adult regimes comprise a daily alovudine dose in the range 2-3 mg/day, such as 2 mg or 2.5 mg. Currently preferred adult regimes have a daily zidovudine dose in the range 450-600 mg, especially 600 mg.

A daily dosage of 2.5 mg alovudine and 600 mg zidovudine corresponds to a molar ratio of 1:218 employing 244 as the molecular weight of alovudine and 269 as the molecular weight of zidovudine.

Co-administration of alovudine and zidovudine at the defined range of ratios may occur sequentially or substantially sequentially, such as when the alovudine and zidovudine are each administered in a separate dosage unit, typically a capsule or a tablet or one of each.

Zidovudine is typically dosed BID, for example 300 mg BID. However, it is often preferable to dose alovudine QD, so a convenient sequentially administered embodiment of the invention could comprise a 300 mg tablet zidovudine and a 2, 3 or 4 mg tablet alovudine in the morning and a 300 mg tablet zidovudine at night (or vice versa). To simplify the pill regime, an alternative, but currently less favoured embodiment could comprise administration of a 300 mg tablet or capsule zidovudine and a 1, 1.5 or 2 mg tablet or capsule containing alovudine, swallowed together or in close succession morning and night.

Sequentially administered dosage forms such as those described in the immediately preceding paragraph may be presented as separate packages, such as respective cartons each containing blister packs of zidovudine tablets or blister packs of alovudine tablets. A further example could comprise separate jars of zidovudine and alovudine capsule or tablets. At least one of the jars or cartons will typically include a package insert or other printed instruction advising that the alovudine is to be co-dosed with zidovudine at the characteristic 1:100 to 1:350 ratio. Conveniently, however, a common carton contains both the blister sheets containing alovudine and the blister sheets containing zidovudine.

In a preferred sequentially administered dosage form the respective alovudine and zidovudine tablets or capsules are presented on the same blister sheet in a spatial arrangement providing visual encouragement of the correct dosing of the respective components. For example if the intended dose of alovudine is 2, 3 or 4 mg QD and the dose of zidovudine is 300 mg BID, the blister sheet may be arranged with one row of alovudine tablets parallel to two rows each with an identical number of zidovudine tablets. It will thus be easy for the patient to ascertain whether or not a given dosing occasion should have both alovudine and zidovudine or zidovudine only. The individual blisters on the blister sheet may be marked with indicia such as the days of the week to further support compliance.

Preferably, however, the alovudine and zidovudine is presented in a common unit dosage form such as a capsule or dragé or more preferably a tablet. The unit dosage form may be adapted for BID administration, i.e. with half the intended daily dose of the alovudine and zidovudine components in each dosage unit, presuming that a single tablet or capsule is administered at any one dosing occasion. A typical unit dosage form in accordance with this embodiment comprises a capsule or tablet containing 300 mg zidovudine and 1 or 1.25 mg alovudine.

If the dosage regime requires multiple, identical dosage units to be ingested at the same (as in the case of the lopinavir/ritonavir combination Kaletra™ which is typically administered as *two* soft tablets each containing 133 mg lopinavir and 33 mg ritonavir three times per day), the amount of alovudine and zidovudine in each unit dose is adjusted accordingly.

An alternative unit dosage form is adapted for QD administration. QD administration facilitates compliance and at the same time minimizes toxicity and provides synergistic antiviral effects. A tablet or capsule intended for adults could thus comprise 2-4 mg alovudine and 300-600 mg zidovudine. Preferred unit dosage forms include:
2 mg alovudine and 300 mg, 400 mg, 500 mg or especially 600 mg zidovudine;
2.5 mg alovudine and 300 mg, 400 mg, 500 mg or especially 600 mg zidovudine;
3 mg alovudine and 300 mg, 400 mg, 500 mg or especially 600 mg zidovudine; or
4 mg alovudine and 300 mg, 400 mg, 500 mg or especially 600 mg zidovudine.

As is common with HIV therapy where combination therapy is the rule rather than the exception, the methods and pharmaceutical compositions of the invention can further comprise one or two additional pharmaceutical agents, in particular additional HIV antivirals. The additional HIV antiviral or antivirals may be taken from any of the mechanistic classes, such as nucleoside reverse transcriptase inhibitors (NRTI), non-nucleoside reverse transcriptase inhibitors (NNRTI), protease inhibitors (PI), integrase inhibitors, fusion inhibitors, maturation inhibitors and the like. The additional HIV antivirals will typically be co-administered or co-dosed at their conventional dosages.

Representative NRTI include stavudine (d4T, Zerit), zalcitabine (ddC), didanosine (ddl, Videx), abacavir, (ABC, Ziagen), lamivudine (3TC, Epivir), emtricitabine (FTC, Emtriva), racevir (racemic FTC), adefovir (ADV), entacavir (BMS 200475), alovudine (FLT), tenofovir disoproxil fumarate (TNF, Viread), amdoxavir (DAPD), D-d4FC (DPC-817), - dOTC (Shire SPD754), elvucitabine (Achillion ACH-126443), BCH 10681 (Shire) SPD-756, racivir, D-FDOC, GS7340, INK-20 (thioether phospholipid AZT, Kucera), 2'3'-dideoxy-3'-fluoroguanosine (FLG) & its prodrugs such as MIV-210, reverset (RVT, D-D4FC, Pharmasset DPC-817).

Representative NNRTI include delavirdine (Rescriptor), efavirenz (DMP-266, Sustiva), nevirapine (BIRG-587, Viramune), (+) calanolide A and B (Advanced Life Sciences), capravirine (AG1549f S-1153; Pfizer), GW-695634 (GW-8248; GSK), MIV-150 (Medivir), MV026048 (MIV-160 Medivir AB), MIV-170 (Medivir) NV-05 2 2 (Idenix Pharm.), R-278474 (Johnson & Johnson), RS-1588 (Idenix Pharm.), TMC-120/125 (Johnson & Johnson), rilpivirine (TMC-278,165335; Johnson & Johnson), UC-781 (Biosyn Inc.) and YM215389 (Yamanoushi).

Representative HIV protease inhibitors include BEA-403 (Medivir) PA-457 (Panacos), KPC-2 (Kucera Pharm.), 5 HGTV-43 (Enzo Biochem), amprenavir (VX-478, Agenerase), atazanavir (Reyataz), indinavir sulfate (MK-639, Crixivan), Lexiva (fosamprenavir calcium, GW -433908 or 908, VX-175), ritonavir (Norvir), lopinavir + ritonavir (ABT-378, Kaletra), tipranavir, nelfinavir mesylate (Viracept), saquinavir (Invirase, Fortovase), AG1776 (JE-2147, KNI-764; Nippon Mining Holdings), AG-1859 (Pfizer), DPC-681/684 (BMS), GS224338; Gilead Sciences), KNI-272 (Nippon Mining Holdings), Nar-DG-35 (Narhex), P(PL)-100 (P-1946; Procyon Biopharma), P-1946 (Procyon Biopharma), R-944 (Hoffmann-LaRoche), RO-0334649 (Hoffmann-LaRoche), TMC-114 (Johnson & Johnson), VX-385 (GW640385; GSK/Vertex), VX-478 (Vertex/GSK).

Other HIV antivirals include entry inhibitors, including fusion inhibitors, inhibitors of the CD4 receptor, inhibitors of the CCR5 co-receptor and inhibitors of the CXCR4 coreceptor, or a pharmaceutically acceptable salt or prodrug thereof. Examples of entry inhibitors are AMD-070 (AMD11070; AnorMed), BlockAide/CR (ADVENTRX Pharm.), BMS 806 (BMS-378806; BMS), Enfurvirtide (T-20, R698, Fuzeon), KRH1636 (Kureha Pharmaceuticals), ONO-4128 (GW-873140, AK-602, E-913; ONO Pharmaceuticals), Pro-140 (Progenics Pharm), PR0542 (Progenics Pharm.), SCH-D (SCH-417690; Schering-Plough), T-1249 (R724; Roche/Trimeris), TAK-220 (Takeda Chem. Ind.), TNX-355 (Tanox) and UK-427,857 (Pfizer). Examples of integrase inhibitors are L-870810 (Merck & Co.), c-2507 (Merck & Co.) and S(RSC)-1838 (Shionogi/GSK).

A currently favoured additional antiviral for use in the methods and pharmaceutical compositions of the invention is MIV-160, also known as *cis-*1-(5-cyanopyridin-2-yl)-3-(4,7-difluoro-1,1a,2,7b-tetrahydrocyclopropa[c]chromen-1-yl)urea. The synthesis of MIV-160 is described in WO02/070516. Suitable adult dosages include 250-1500mg, such as 400 or 800 mg, typically QD or BID.

The embodiments of the invention comprising MIV-160 are conveniently co-dosed with a cytochrome antagonist, especially a Cyp450 3A4 inhibitor such as grape fruit juice or more preferably ritonavir. Ritonavir is a protease inhibitor already registered for the treatment of HIV with a recommended adult dose of 600 mg BID, but a much lower dose, typically 100 or 200 mg BID, when used as a booster. Use of a booster typically allows the MIV-160 dose to be reduced.

Typical unit dosage embodiments for this aspect of the invention include tablets comprising:

| **alovudine** | **zidovudine** | **MIV-160** | **ritonavir** | **regime** |
|---|---|---|---|---|
| 1 mg | 300 mg | 800mg | | BID |
| 1 mg | 300 mg | 100 mg | 100 mg | BID |
| 1.5 mg | 300 mg | 800 mg | | BID |
| 1.5 mg | 300 mg | 100 mg | 100 mg | BID |
| 2 mg | 600 mg | 1500 mg | | QD |
| 2 mg | 300 mg | 100 mg | 100 mg | QD |
| 2 mg | 300 mg | 200 mg | 100 mg | QD |
| 2mg | 600 mg | 100 mg | 100 mg | QD |
| 2 mg | 600 mg | 200 mg | 100 mg | QD |
| 4 mg | 400 mg | 200mg | 100 mg | QD |

| **QD** | **BID** | | | |
|---|---|---|---|---|
| 2 mg | 300 mg | 100 mg | 100 mg | |
| 2mg | 300 mg | 200 mg | 100 mg | |
| 4mg | 300mg | 200 mg | 100 mg | |

| **QD** | **BID** | **QD** | **QD** | |
|---|---|---|---|---|
| 2 mg | 300 mg | 100 mg | 100 mg | |
| 2 mg | 300 mg | 200 mg | 100 mg | |
| 4mg | 300 mg | 200 mg | 100 mg | |

A further favoured NNRTI for use in the invention is MIV-170, otherwise known as N-[(1S,1aR,7bR)-4,7-difluoro-1,1a,2,7b-tetrahydrocyclopropa[c]chromen-1-yl]-N'-[5-(4-(sulfonamido)phenoxy)-2-pyridinyl]urea. The synthesis of MIV-170 is shown in WO05/066131. Typical adult dosages are of the order of 100-900 mg/day, especially 300-600 mg QD. MIV-170 combinations with alovudine and zidovudine at the characteristic ratio will generally not need to be ritonavir boosted due to substantially improved oral bioavailability.

Typical regimes of this embodiment include:

| **alovudine** | **zidovudine** | **MIV-170** | **regime** |
|---|---|---|---|
| 1 mg | 300 mg | 300 mg | BID |
| 1.5 mg | 300 mg | 300 mg | BID |
| 2 mg | 600 mg | 600 mg | QD |
| 2 mg | 600 mg | 900 mg | QD |
| 4 mg | 400 mg | 600 mg | QD |
| 2 mg QD | 300 mg BID | 300 mg BID | |
| 2 mg QD | 300 mg BID | 600 mg QD | |
| 2 mg QD | 300 mg BID | 900 mg QD | |

Returning now to the invention in general, alovudine and zidovudine are not generally regarded as difficult to formulate and conventional galenic methods, excipients and carriers are widely available. Co-formulation of alovudine and zidovudine with any of the above mentioned additional antivirals may require adoption of formulations appropriate for that additional antiviral as will be readily apparent to the skilled practitioner. The preferred additional antivirals MIV-160, MIV-170 and ritonavir are not known to pose insurmountable challenges to formulation.

Such well known galenic methods include the step of bringing alovudine and zidovudine in the specified characteristic range of ratios, and any additional antiviral, into association with a conventional pharmaceutical carrier. In general, the formulations are prepared by uniformly and intimately bringing the active agents into association with liquid carriers or finely divided solid carriers or both, and then shaping the product, if necessary. The invention extends to methods for preparing a pharmaceutical composition comprising bringing alovudine and zidovudine in the specified characterstic range of ratios, and optionally one or two additional antivirals, in conjunction or association with a pharmaceutically acceptable carrier or vehicle. If the manufacture of pharmaceutical formulations involves intimate mixing of pharmaceutical excipients and the active ingredient is in a salt form, then it is often preferred to use excipients which are non-basic in nature, i.e. either acidic or neutral.

The formulations for oral administration of the present invention may be presented as discrete units such as capsules, cachets or tablets, each containing a predetermined amount of the active agents. Alternatively they can be presented as a powder or granules; as a solution or a suspension of the active agent in an aqueous liquid or a non-aqueous liquid, or as an oil-in-water liquid emulsion or a water-in-oil liquid emulsion, as a bolus, etc.

With regard to compositions for oral administration (e.g. tablets and capsules), the term "suitable carrier" includes vehicles such as common excipients, for example binding agents such as syrup, acacia, gelatin, sorbitol, tragacanth, polyvinylpyrrolidone (Povidone), methylcellulose, ethylcellulose, sodium carboxymethylcellulose, hydroxypropyl-methylcellulose, sucrose and starch; fillers and carriers, for example corn starch, gelatin, lactose, sucrose, microcrystalline cellulose, kaolin, mannitol, dicalcium phosphate, sodium chloride and alginic acid; and lubricants such as magnesium stearate, sodium stearate and other metallic stearates, glycerol stearate stearic acid, silicone fluid, talc waxes, oils and colloidal silica. Flavouring agents such as peppermint, oil of wintergreen, cherry flavouring or the like can also be used. It may be desirable to add a colouring agent to make the dosage form readily identifiable. Tablets may also be coated by methods well known in the art.

A tablet may be made by compression or molding, optionally with one or more accessory ingredient. Compressed tablets may be prepared by compressing in a suitable machine the active agent in a free flowing form such as a powder or granules, optionally mixed with a binder, lubricant, inert diluent, preservative, surface-active or dispersing agent. Molded tablets may be made by molding in a suitable machine a mixture of the powdered compound moistened with an inert liquid diluent. The tablets may optionally be coated or scored and may be formulated so as to provide slow or controlled release of the active agent.

Other formulations suitable for oral administration include lozenges comprising the active agent in a flavoured base, usually sucrose and acacia or tragacanth; pastilles comprising the active agent in an inert base such as gelatin and glycerin, or sucrose and acacia; and mouthwashes comprising the active agent in a suitable liquid carrier.

Alovudine and/or zidovudine may be dosed as the free nucleoside or a conventional pharmaceutically acceptable salt or hydrate thereof. Conventional salts include acid addition salts such as hydrochloride, hydrobromide, citrate, tosylate and maleate salts and salts formed with phosphoric or sulphuric acid. In another aspect suitable salts are base salts such as an alkali metal salt for example sodium or potassium, an alkaline earth metal salt for example calcium or magnesium, or organic amine salt for example triethylamine. Examples of solvates include hydrates.

Alternatively alovudine and/or zidovudine may be dosed as a prodrug which releases alovudine/zidovudine or alovudine/zidovudine monophosphate in vivo. Conventional nucleoside prodrugs releasing the nucleoside in vivo include 5'-alkyl esters such as the acetyl, pivaloyl or stearoyl or 5'amino esters such as the L-valyl, L-isoleucyl or L-lactyl-L-valyl esters. Prodrugs releasing zidovudine monophosphate in vivo include fosivudine, such as fosivudine tidoxil. Prodrugs releasing alovudine in vivo include the fosivudine analogues described in EP 350 287, EP 545 966, EP741 740 & EP763 049, such as fosalvudine tidoxil: 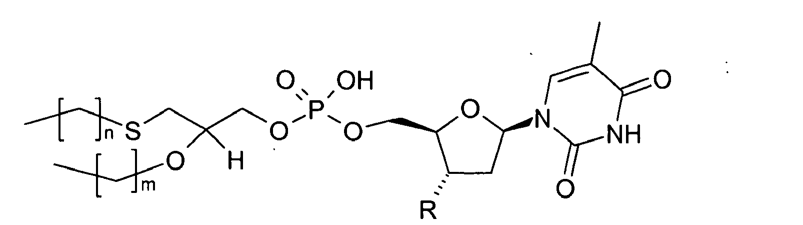 where n is 11, m is 9 and R is F (fosalvudine) or N₃ (fosivudine).

References to alovudine and zidovudine in this specification and claims refer also to such salts, hydrate and prodrugs, wherein the weight amounts (such as daily doses) are typically adjusted upward to correspond with the increased molecular weight relative to the free nucleoside.

Zidovudine is now generic and is widely available in pharmaceutical grade from many manufacturers around the world. Alovudine is conveniently synthesized using the aluminium or iron catalsyed anhydronucleoside routes described in or analogous to EP 470 355 or WO 94/26762. Fosivudine and fosalvudine are prepared in the patents cited above. The synthesis of MIV-160 and MIV-170 is as specified above.

### Detailed description of the embodiments

Various aspects of the invention will now be described by way of illustration only with reference to the following non-limiting examples.

### Mitochondrial toxicity determined in cell line experiments.

Experiments are described below designed to illuminate inhibition of mitochondrial DNA synthesis following administration of alovudine, zidovudine or both. Several suitable cell lines supporting HIV growth are readily available including lines include those supporting HIV growth including the CEMx174 cell line derived from Swedish Institute for Infectious Disease Control (SMI) Sweden, MT-4 (commercially available) and Hep G2 (commercially available).

In short, 100 ul cells are seeded in a 96-well plate at a concentration of 1x10⁴ cells/ml, at exponential growth were cultured in RPMI 1640 medium (from Gibco) supplemented with 10% heat-inactivated fetal bovine serum (from Gibco) and Penicillin-Streptomycin (from Gibco). The medium is changed every 3 or 4 days, and cells are subcultured once a week at a dilution of 1:10. All cultures are routinely checked for Mycoplasma infection and grown at 37°C in a humidified 5% CO₂ atmosphere.

All drugs tested were first dissolved at 10 mM in dimethyl sulfoxide (DMSO) before further dilution to the appropriate concentration in the culture medium. The analysis of mitochondrial DNA was performed by using Taqman technology as previously reported (Zhang, H et al. Mol. Pharmacol 1994 46:1063-1069), with modifications, and is briefly described below.

The cells are treated for a series of durations with the test drugs, such as alovudine, fosalvudine, zidovudine, abacavir etc or various combinations of alovudine/fosalvudine and zidovudine/abacavir. The various ratios indicated in the respective Tables. After 14 days of drug exposure, cells are collected. Total cellular DNA was prepared using the QiAamp DNA blood Mini kit (QIAGEN, Chatsworth, CA), following the supplier's instructions for the Qiagene Blood&Body Fluid Spin Protocol, and subject to DNA amplification.

The probes of mitochondrial DNA Taqman and human nuclear DNA were prepared from Applied Biosystems, which employs an internal quenched DNA probe utilizing fluorescence resonance energy transfer (FRET) to generate a spectroscopic response due to 5'-->3' exonuclease activity of Taq DNA polymerase during DNA amplification. This process uses a PCR-based assay with laser scanning technology to excite fluorescent dyes present in a specially designed TaqMan probes: Mitochondrial DNA probe corresponding to D-loop, 5'Fluoro Label, 6-FAM-ACG CTG GAG CCG GAG-MGBNFQ; Probes for nuclear DNA corresponding to the 18S ribosomal RNA, 5'FLUOR Label, 6'FAM-TCG AAC GTC TGC CC-MGBNFQ together with a pair of DNA amplification primers (forward mitochondrial DNA primer: 5'-CAC GCG ATA GCA TTG CGA-3' and reverse mitochondrial DNA primer: 5'-AGG AAT CAA AGA CAG ATA CTG CGA-3'. Forward nuclear DNA primer: 5'-GCG GCG ACG ACC CA-3' and reverse cellular nuclear DNA primer: 5'-GGC GAC TAC CAT CGA AAG TTG-3'). It is a fully integrated system for real-time detection of PCR using ABI PRISM 7700 and TaqMan reagents for the fluorogenic 5' nuclease assay.

The cell growth was controlled by cellular nuclear DNA (16S ribosomal DNA). The result from the mitochondrial assay is presented as the percentage inhibition of mitochondrial DNA and cellular nuclear DNA compared to the control (without drug exposure).

### Example 1

The above described assay was performed in the MT4 cell line derived from a T lymphocyte. Monotherapy with alovudine or zidovudine or combination therapy with combinations of alovudine & zidovudine were performed at the molar concentrations reported in Table 1 below:

**Table 1**

| **Treatment** | **Inhibition of mitochondrial DNA** |
|---|---|
| | % |
| 0.1 uM alovudine | 70 |
| 0.3 uM alovudine | 95 |
| 1 uM alovudine | 100 |
| 1 uM zidovudine | 0 |
| 3 uM zidovudine | 0 |
| 10 uM zidovudine | 0 |
| 0.01 uM alovudine + 1 uM zidovudine | 0 |
| 0.03 uM alovudine + 3 uM zidovudine | 0 |
| 0.1 uM alovudine + 10 uM zidovudine | 0 |
| 1 uM alovudine + 10 uM zidovudine | 60% reduction in nuclear DNA and |
| | 30% reduction in mitochondrial DNA |

It will be apparent from Table 1 that monotherapy with alovudine, even in concentrations as low as 0.1 uM induced a 70% reduction of mitochondrial DNA copy number in this experiment. This inhibition is eliminated by the presence of zidovudine at a ratio 1:100 in molar terms.

Looking now at the combination 1 uM alovudine:10 uM zidovudine, a ratio within the particularly preferred range of the above mentioned WO91/01137, it is noted that there is considerable cellular toxicity (as reflected by a 60% drop in nuclear DNA copy number) together with a substantial drop in mitochondrial DNA copy number. In clinical terms this cellular toxicity masks the decreased mitochondrial toxicity relative to alovudine alone. However, the point is that a very appreciable level of mitochondrial toxicity remains in this prior art alovudine/zidovudine combination.

In contrast, alovudine and zidovudine at a molar ratio of 1:100 (corresponding to 1:110 on a wt:wt basis) within the scope of the invention completely abolishes the reduction in mitochondrial DNA copy number.

### Example 2

The above experiment was repeated in MT-4 cells with additional concentrations of alovurdine and/or zidovudine. Raw and statistical data are presented in Table 2A. The data are as summarized in Table 2B below:

**Table 2A**

| **Treatment** | **Relative no. copies** | | | | **% reduction** | |
|---|---|---|---|---|---|---|
| | **mt DNA** | **SD** | **n DNA** | **SD** | **mt DNA** | **n DNA** |
| zidovudine 60 uM | 748730 | 116013 | 890663 | 89057 | 4 | 40 |
| zidovudine 30 uM | 993321 | 133615 | 836935 | 257217 | 0 | 43 |
| zidovudine 10 uM | 856782 | 100863 | 891446 | 242554 | 0 | 40 |
| alovudine 0.6 uM | 96998 | 13914 | 1740000 | 889132 | 88 | 0 |
| alovudine 0.3 uM | 247481 | 36417 | 1160000 | 158760 | 68 | 22 |
| alovudine 0.1 uM | 434709 | 17528 | 956331 | 199387 | 44 | 35 |
| 0.6 uM alovudine + 60 uM zidovudine | 520937 | 117899 | 303007 | 32122 | 33 | 80 |
| 0.3 uM alovudine + 30 uM zidovudine | 985449 | 79005 | 1380000 | 307833 | 0 | 7 |
| 0.1 uM alovudine + 10 uM zidovudine | 758191 | 65015 | 1790000 | 772598 | 2 | 0 |
| Untreated cells | 777492 | 287608 | 1480000 | 97906 | 0 | 0 |

**Table 2B**

| **Treatment** | **Ratio Mitochondrial: Nuclear DNA** | **% Reduction mt/n DNA** |
|---|---|---|
| zidovudine 60 uM | 0.84 | 0 |
| zidovudine 30 uM | 1.19 | 0 |
| zidovudine 10 uM | 0.96 | 0 |
| alovudine 0.6 uM | 0.06 | 89 |
| alovudine 0.3 uM | 0.21 | 59 |
| alovudine 0.1 uM | 0.45 | 13 |
| 0.6 uM alovudine + 60 uM zidovudine | 1.72 | 0 |
| 0.3 uM alovudine + 30 uM zidovudine | 0.71 | 0 |
| 0.1 uM alovudine + 10 uM zidovudine | 0.42 | 19 |
| Untreated cells | 0.53 | 0 |

The experiment thus confirms Example 1 that the mitochondrial toxicity of alovudine can be reversed by the co-administration of a significantly larger molar concentration of zidovudine.

### Example 3

A further mitochondrial depletion assay was carried out in MT-4 cells, employing the alovudine monophosphate prodrug fosalvudine tidoxil, synthesized as described in example 19 of EP741740 and/or zidovudine. The results are shown in Table 3

**Table 3**

| **Treatment** | **Mean qty** | **SD** | **Inhibition of mitochondrial DNA, %** |
|---|---|---|---|
| 0.3 uM fosalvudine tidoxil | 507917 | 133430 | 35 |
| 30 uM zidovudine | 1410000 | 295912 | 0 |
| 0.3 uM fosalvudine tidoxil + 30 uM zidovudine | 1040000 | 115835 | 0 |
| Untreated cells | 771791 | 206722 | 0 |

Again, the alovudine, (n this case administered as the monophosphate prodrug), in monotherapy was associated with significant mitochondrial toxicity, as measured by mt DNA depletion. This toxicity was reversed by co-administration of a 100-fold greater concentration of zidovudine.

### Comparative Example 1

Mitochondrial toxicity of alovudine vs alovudine plus abacavir determined in MT-4 cells.

Abacavir and alovudine at a molar ratio of 70:1 have exhibited synergistic activity in a phase II clinical trial - see W02004/002433. This patent application also describes synergistic activity as regards antiviral affect in a cell culture assay at a molar ratio of 200:1. Abacavir, alovudine or abacavir & alovudine at various concentrations were tested as described in Example 1:

**Table 4**

| **Treatment** | **Mean qty mt DNA** | **SD** | **Inhibition of mitochondrial DNA, %** |
|---|---|---|---|
| 0.1 uM alovudine | 549900 | 214665 | 40 |
| 1 uM abacavir | 756544 | 67855 | 18 |
| 0.1 uM alovudine + 1 uM abacavir | 489326 | 144267 | 48 |
| Untreated cells | 771791 | 206722 | 0 |
| | | | |
| 0.2 uM alovudine | 187574 | 124354 | 82 |
| 20 uM abacavir | 506181 | 404843 | 51 |
| 0.2 uM alovudine + 20 uM abacavir | 226201 | 173273 | 78 |
| Untreated cells | 1030000 | 480333 | 0 |

The results show that the mitochondrial toxicity of alovudine was not reversed by addition of the NRTI abacavir at low or high molar ratios (1:10 alovudine:abacavir or 1:100 alovudine or abacavir). This implies that the synergy being achieved as regards antiviral efficacy has no mechanistic link with antagonism as regards mitochondrial toxity.

Throughout the specification and the claims which follow, unless the context requires otherwise, the word 'comprise', and variations such as 'comprises' and 'comprising', will be understood to imply the inclusion of a stated integer or step or group of integers but not to the exclusion of any other integer or step or group of integers or steps.

## Claims

1. Alovudine for use in the treatment or prevention of HIV with simultaneous or sequential administration of zidovudine at a molar ratio of alovudine to zidovudine in the range 1:100 to 1:350.

2. Alovudine according to claim 1, for use with zidovudine at a molar ratio of alovudine to zidovudine in the range 1:150 to 1:250, preferably 1:150 to 1:200.

3. Alovudine according to either claim 1 or claim 2, wherein the alovudine dosage is in the range 2-4 mg per day and the zidovudine dosage is in the rage 300-900 mg per day.

4. Alovudine according to any one of claims 1 to 3, wherein the dose of alovudine is 2-4 mg QD and zidovudine 300 mg BID.

5. Alovudine according to any one of claims 1 to 3, wherein the alovudine is administered in a QD unit dosage form.

6. Zidovudine for use in the treatment or prevention of HIV with simultaneous or sequential administration of alovudine at a molar ratio of alovudine to zidovudine in the range 1:100 to 1:350.

7. Zidovudine according to claim 6, for use with alovudine at a molar ratio of alovudine to zidovudine in the range 1:150 to 1:250, preferably 1:150 to 1:200.

8. Zidovudine according to either claim 6 or claim 7, wherein the alovudine dosage is in the range 2-4 mg per day and the zidovudine dosage is in the rage 300-900 mg per day.

9. Zidovudine according to any one of claims 6 to 8, wherein the dose of alovudine is 2-4 mg QD and zidovudine 300 mg BID.

10. Use of alovudine and zidovudine in the manufacture of medicaments for simultaneous or sequential administration, whereby the medicaments are adapted to encourage dosing at a molar ratio of alovudine to zidovudine in the range 1:100 to 1:350.

11. A pharmaceutical composition comprising alovudine and zidovudine in a molar ratio in the range 1:100 to 1:350 and wherein the alovudine is present in the range 2-4 mg and the zidovdine is present in the range 300-900 mg.

12. A pharmaceutical composition according to claim 11 wherein the ratio is in the range 1:150 to 1:250, preferably 1:150 to 1:200.

13. A pharmaceutical composition according to either claim 11 or 12, further comprising one or two additional pharmaceutical agents.

14. A pharmaceutical composition according to any of claims 11 to 13, presented in a QD unit dosage form.

15. A kit of parts comprising:
(i) a pharmaceutical composition comprising alovudine; and
(ii) a pharmaceutical composition comprising zidovudine;
**characterized in that that** the alovudine and zidovudine are present in the kit at a molar ratio in the range 1:100 to 1:350.

## Patentansprüche

1. Alovudin zur Verwendung bei der Behandlung oder Prävention von HIV mit gleichzeitiger oder aufeinanderfolgender Verabreichung von Zidovudin in einem Alovudin:Zidovudin-Molverhältnis im Bereich von 1:100 bis 1:350.

2. Alovudin nach Anspruch 1 zur Verwendung mit Zidovudin in einem Alovudin:Zidovudin-Molverhältnis im Bereich von 1:150 bis 1:250, vorzugsweise 1:150 bis 1:200.

3. Alovudin nach Anspruch 1 oder 2, wobei die Alovudindosierung im Bereich von 2-4 mg pro Tag und die Zidovudindosierung im Bereich von 300-900 mg pro Tag liegt.

4. Alovudin nach einem der Ansprüche 1 bis 3, wobei die Alovudindosis 2-4 mg QD und die Zidovudindosis 300 mg BID beträgt.

5. Alovudin nach einem der Ansprüche 1 bis 3, wobei das Alovudin in einer Q D-Einzeldosisform verabreicht wird.

6. Zidovudin zur Verwendung bei der Behandlung oder Prävention von HIV mit gleichzeitiger oder aufeinanderfolgender Verabreichung von Alovudin in einem Alovudin:Zidovudin-Molverhältnis im Bereich von 1:100 bis 1:350.

7. Zidovudin nach Anspruch 6 zur Verwendung mit Alovudin in einem Alovudin:Zidovudin-Molverhältnis im Bereich von 1:150 bis 1:250, vorzugsweise 1:150 bis 1:200.

8. Zidovudin nach Anspruch 6 oder 7, wobei die Alovudindosierung im Bereich von 2-4 mg pro Tag und die Zidovudindosierung im Bereich von 300-900 mg pro Tag liegt.

9. Zidovudin nach einem der Ansprüche 6 bis 8, wobei die Alovudindosis 2-4 mg QD und die Zidovudindosis 300 mg BID beträgt.

10. Verwendung von Alovudin und Zidovudin bei der Herstellung von Medikamenten zur gleichzeitigen oder aufeinanderfolgenden Verabreichung, wobei die Medikamente so eingestellt sind, dass eine Verabreichung in einem Alovudin:Zidovudin-Molverhältnis im Bereich von 1:100 bis 1:350 begünstigt wird.

11. Pharmazeutische Zusammensetzung, enthaltend Alovudin und Zidovudin in einem Molverhältnis im Bereich von 1:100 bis 1:350, wobei das Alovudin im Bereich von 2-4 mg vorhanden ist und das Zidovudin im Bereich von 300-900 mg vorhanden ist.

12. Pharmazeutische Zusammensetzung nach Anspruch 11, wobei das Verhältnis im Bereich von 1:150 bis 1:250, vorzugsweise 1:150 bis 1:200, liegt.

13. Pharmazeutische Zusammensetzung nach Anspruch 11 oder 12, weiterhin enthaltend ein oder zwei zusätzliche pharmazeutische Mittel.

14. Pharmazeutische Zusammensetzung nach einem der Ansprüche 11 bis 13, welche in einer QD-Einheitsdosisform vorliegt.

15. Kit aus Teilen, umfassend:
(i) eine Alovudin enthaltende pharmazeutische Zusammensetzung und
(ii) eine Zidovudin enthaltende pharmazeutische Zusammensetzung;
**dadurch gekennzeichnet, dass** das Alovudin und das Zidovudin in dem Kit in einem Molverhältnis im Bereich von 1:100 bis 1:350 vorliegen.

## Revendications

1. Alovudine pour application au traitement prophylactique ou thérapeutique du VIH avec administration simultanée ou séquentielle de zidovudine dans un rapport molaire alovudine sur zidovudine compris dans l'intervalle 1:100 à 1:350.

2. Alovudine conforme à la revendication 1, pour emploi avec la zidovudine dans un rapport molaire alovudine sur zidovudine compris dans l'intervalle 1:150 à 1:250, préférentiellement 1:150 à 1:200.

3. Alovudine conforme à la revendication 1 ou à la revendication 2, où la dose d'alovudine est comprise dans l'intervalle 2 à 4 mg par jour et la dose de zidovudine est comprise dans l'intervalle 300 à 900 mg par jour.

4. Alovudine conforme à l'une quelconque des revendications 1 à 3, où la dose d'alovudine est de 2 à 4 mg une fois par jour et la dose de zidovudine est de 300 mg deux fois par jour.

5. Alovudine conforme à l'une quelconque des revendications 1 à 3, où l'alovudine est administrée sous une forme galénique unitaire une fois par jour.

6. Zidovudine pour application au traitement prophylactique ou thérapeutique du VIH avec administration simultanée ou séquentielle d'alovudine dans un rapport molaire alovudine sur zidovudine compris dans l'intervalle 1:100 à 1:350.

7. Zidovudine conforme à la revendication 6, pour emploi avec l'alovudine dans un rapport molaire alovudine sur zidovudine compris dans l'intervalle 1:150 à 1:250, préférentiellement 1:150 à 1:200.

8. Zidovudine conforme à la revendication 6 ou à la revendication 7, où la dose d'alovudine est comprise dans l'intervalle 2 à 4 mg par jour et la dose de zidovudine est comprise dans l'intervalle 300 à 900 mg par jour.

9. Zidovudine conforme à l'une quelconque des revendications 6 à 8, où la dose d'alovudine est de 2 à 4 mg une fois par jour et la dose de zidovudine est de 300 mg deux fois par jour.

10. Emploi d'alovudine et de zidovudine dans la fabrication de médicaments pour administration simultanée ou séquentielle, les médicaments étant fabriqués de sorte à encourager l'administration de doses dans un rapport molaire alovudine sur zidovudine compris dans l'intervalle 1:100 à 1:350.

11. Composition pharmaceutique comprenant de l'alovudine et de la zidovudine dans un rapport molaire compris dans l'intervalle 1:100 à 1:350 et où l'alovudine est présente dans une quantité comprise dans l'intervalle 2 à 4 mg et la zidovudine est présente dans une quantité comprise dans l'intervalle 300 à 900 mg.

12. Composition pharmaceutique conforme à la revendication 11 où le rapport est compris dans l'intervalle 1:150 à 1:250, préférentiellement 1:150 à 1:200.

13. Composition pharmaceutique conforme à la revendication 11 ou 12, comprenant en outre un ou deux agents pharmaceutiques supplémentaires.

14. Composition pharmaceutique conforme à l'une quelconque des revendications 11 à 13, se présentant sous une forme galénique unitaire pour administration une fois par jour.

15. Kit d'éléments comprenant :
(i) une composition pharmaceutique comprenant de l'alovudine ; et
(ii) une composition pharmaceutique comprenant de la zidovudine ;
**caractérisé en ce que** l'alovudine et la zidovudine sont présentes dans le kit dans un rapport molaire compris dans l'intervalle 1:100 à 1:350.
